(19) Europäisches Patentamt
European Patent Office
Office européen des brevets

(11) **EP 4 550 178 A1**

(12) **EUROPEAN PATENT APPLICATION**
published in accordance with Art. 153(4) EPC

(43) Date of publication:
**07.05.2025 Bulletin 2025/19**

(21) Application number: **23916584.8**

(22) Date of filing: **20.12.2023**

(51) International Patent Classification (IPC):
**G06F 17/10** (2006.01)  **G06T 7/20** (2017.01)
**G06F 17/16** (2006.01)  **A61H 7/00** (2006.01)

(52) Cooperative Patent Classification (CPC):
Y02P 90/30

(86) International application number:
**PCT/CN2023/140192**

(87) International publication number:
**WO 2025/060284 (27.03.2025 Gazette 2025/13)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **18.09.2023  CN 202311200597**

(71) Applicant: **Realman Intelligent Technology
(Beijing) Co., Ltd.
Beijing 100043 (CN)**

(72) Inventors:
• LAN, Hui
  **Beijing 100043 (CN)**
• ZHANG, Ying
  **Beijing 100043 (CN)**
• DONG, Qinpeng
  **Beijing 100043 (CN)**
• ZHANG, Xulong
  **Beijing 100043 (CN)**
• ZHENG, Suibing
  **Beijing 100043 (CN)**

(74) Representative: **Huang, Liwei
  Cäcilienstraße 12
  40597 Düsseldorf (DE)**

(54) **MASSAGE PATH GENERATION METHOD AND SYSTEM**

(57)   A method and system for generating a massage track, including acquiring two-dimensional data of preliminary massage-point positions according to a user-selected massage mode; acquiring point cloud data of a region to be massaged in a target object; acquiring the massage point position two-dimensional data of the target by proportion calculation according to the preliminary massage-point position two-dimensional data and the point cloud data of the region to be massaged in the target object; acquiring target three-dimensional massage point position data according to the two-dimensional data of target massage-point positions and the point cloud data of the region to be massaged in the target object; and generating a final massage track based on a preset massage track planning algorithm according to the target three-dimensional massage point position data. The present invention may select a massage mode to improve individual adaptability. According to the individual differences, the two-dimensional data of the massage point positions of the target are obtained to further improve the individual adaptability, and also provide a basis for further massage track planning. The preset massage track planning algorithm reduces the amount of calculation and the planned massage track is smooth, which is conducive to promotion.

FIG. 1

## Description

### Cross-reference

The present application is based on and claims the priority of the Chinese patent application with the application number of 202311200597X and the filing date of 18 September 2023, and the whole content of the Chinese patent application is incorporated herein by reference.

### Technical Field

The present invention relates to the field of robotic massage technology, and in particular to a method and system for generating a massage track.

### Background Art

Massage tracks of massage equipment such as automatic massage devices such as massage chairs and massage beds are preset and can only provide a user with a massage according to a fixed massage track. The body type of client and the position where the target object lies will affect the massage effect, and the adaptability for different users is poor.

The physical interaction between robot and human is an emerging research field in recent years. Machine vision has developed rapidly in recent years. Many universities and research institutions combine the technology of machine vision with the massage robot to promote the further development of massage robot. Nowadays, with the development of computer technology, the modem intelligent robot technology has become more and more important. With the help of new technologies such as perception/Internet/cloud storage, the massage robot can help or replace the massager's action of conventional massage.

With the advent of massage robots, there is a tendency to plan massage tracks independently for different users. However, the tracks of the existing massage robot planning are mainly linear tracks with a single form, and the contour of the target object is poorly adapted. Based on the curve trajectory of Bessel curve planning, the spiral trajectory is limited by the characteristics of Bessel curve. The change of a massage point will affect the shape of the whole massage track, and the number of massage-point positions is directly related to the order of Bessel curve. When the point increases, the calculation difficulty of Bessel curve will be increased, which is a challenge to the computing power of massage robot.

### Summary of the Invention

(I) Objects of the invention

It is an object of the present invention to provide a method and system for generating a massage track that reduce the amount of calculation, improve the individual adaptability, and smooth the massage track.

(II) Technical solution

In order to solve the above problems, the present invention provides a method for generating a massage track, comprising:

acquiring two-dimensional data of preliminary massage-point positions according to a selected massage mode;
acquiring point cloud data of a region to be massaged in a target object;
determining a proportion coefficient according to the two-dimensional data of the preliminary massage-point position and the point cloud data of the region to be massaged in the target object, and determining the two-dimensional data of target massage-point positions based on the two-dimensional data of the preliminary massage-point positions and the proportion coefficient;
acquiring target three-dimensional massage point position data according to the two-dimensional data of target massage-point positions and the point cloud data of the region to be massaged in the target object; and
generating a final massage track based on a preset massage track planning algorithm according to the target three-dimensional massage point position data;
wherein the preset massage track planning algorithm comprises grouping target three-dimensional massage-point positions according to a preset grouping rule to obtain a plurality of massage point position groups;
generating a plurality of segments of massage tracks according to the plurality of massage point position groups; and
splicing the plurality of segments of massage tracks to generate a final massage track.

In another aspect of the present invention, it is preferable that the generating a final massage track based on a preset massage track planning algorithm according to the target three-dimensional massage point position data further comprises:

selecting key points of massage point positions in a plurality of massage point position groups respectively;
calculating node vectors of a plurality of massage point position groups respectively, based on the key points according to a preset first calculation method;
presetting boundary conditions of a plurality of massage point position groups;
according to the node vector and the preset boundary condition, calculating control vertices of the plurality of massage point position groups respectively, according to a preset second calculation method;
generating a plurality of segments of massage tracks according to the control vertices of the plurality of massage point position groups according to a preset third calculation method; and
splicing the plurality of segments of massage tracks to generate a preliminary massage track.

In another aspect of the present invention, it is preferable that the generating a final massage track based on a preset massage track planning algorithm according to the target three-dimensional massage point position data further comprises:
according to the target three-dimensional massage point position data in the preliminary massage track, calculating the pose of each target three-dimensional massage point position according to a preset pose algorithm, and generating a final massage track.

In another aspect of the present invention, preferably, the preset grouping rule comprises:

acquiring the number of massag-point positions according to the target three-dimensional massage point position data;
presetting a minimum number of the grouping units;
judging whether the number of massage-point positions is greater than a preset minimum number of grouping units;
if the number of massage-point positions is greater than a preset minimum number of grouping units, successively sliding a frame to take points for grouping according to the preset minimum number of grouping units until the number of massage-point positions of the last group is less than or equal to the preset minimum number of grouping units; and
if the number of massage-point positions is less than or equal to the preset minimum number of grouping units, no grouping is required.

In another aspect of the present invention, it is preferable that the preset first calculation method includes:

presetting a first-end node repetition degree to 4;

$$u_0 = u_1 = u_2 = u_3 = 0$$

$$u_{i+3} = u_{i+2} + \frac{|p_i - p_{i-1}|}{\sum_{t=1}^{n}|p_t - p_{t-1}|}$$

$$u_{n+2} = u_{n+3} = u_{n+4} = u_{n+5} = 1$$

wherein the node vector is represented as $U = [u_0, u_1, ..., u_{n+5}]$; n represents the number of key points; i represents a sequence number of the current node; $p_i$: an $i^{th}$ key point; t: a sequence number of summation of all key points; and pt: a $t^{th}$ key point.

In another aspect of the present invention, it is preferable that the preset second calculation method includes:

obtaining a control vertex by calculating an inverse matrix of a following square matrix;

$$\begin{bmatrix} 1 & & & & & \\ a_2 & b_2 & c_2 & & & \\ & \bullet & \bullet & \bullet & & \\ & & \bullet & \bullet & \bullet & \\ & & & \bullet & \bullet & \bullet \\ & & & a_{n-1} & b_{n-1} & c_{n-1} \\ & & & & & 1 \end{bmatrix} \begin{bmatrix} d_1 \\ d_2 \\ \bullet \\ \bullet \\ \bullet \\ d_{n-1} \\ d_n \end{bmatrix} = \begin{bmatrix} e_1 \\ e_2 \\ \bullet \\ \bullet \\ \bullet \\ e_{n-1} \\ e_n \end{bmatrix}$$

where $d_i$ is a control vertex; and $a_i, b_i, c_i, e_i$ are all parameters related to key points and node vectors.

In another aspect of the present invention, it is preferable that the preset third calculation method includes:

$$p(u) = \frac{\sum_{i=0}^{n} \omega_i d_i N_{i,k}(u)}{\sum_{i=0}^{n} \omega_i N_{i,k}(u)}$$

where u represents a node parameter and an argument $\in [0, 1]$; $d_i$ represents a control vertex; k represents the order of a curve; $N_{i,k}(u)$ represents a $k^{th}$ B-spline fundamental function; and $\omega_i$ represents a weight factor.

In another aspect of the present invention, preferably, the B-spline fundamental function formulation comprises:

$$N_{i,0}(u) = \begin{cases} 1, & u_i \le u < u_{i+1} \\ 0, & \text{others} \end{cases}$$

$$N_{i,k}(u) = \frac{u - u_i}{u_{i+k} - u_i} N_{i,k-1}(u) + \frac{u_{i+k+1} - u}{u_{i+k+1} - u_{i+1}} N_{i+1,k-1}(u)$$

Setting

$$\frac{0}{0} = 0$$

where $N_{i,k}(u)$ represents a $k^{th}$ B-spline fundamental function; and u represents a node parameter.

In another aspect of the present invention, preferably, the preset pose algorithm includes:

according to the target three-dimensional massage point position data, the three-dimensional massage point position data comprises a three-dimensional coordinate value and a Z-axis direction vector of the massage point position; the three-dimensional coordinate value comprises X-axis, Y-axis and Z-axis data;
acquiring an X-axis direction vector of each three-dimensional massage point position of the target according to data of each target three-dimensional massage point position in the preliminary massage track;
acquiring a Y-axis direction vector according to a cross product of the X-axis and Z-axis direction vectors of each target three-dimensional massage point positions;
correcting the Z-axis direction vector by using a three-axis pair-wise perpendicular manner according to the direction vectors of the X-axis and Y-axis; and
obtaining the pose of each target three-dimensional massage point position, and generating a final massage track.

In another aspect of the present invention, preferably, a system for generating a massage track includes:

a selecting module configured for acquiring two-dimensional data of preliminary massage-point positions according to a selected massage mode;
an acquisition module configured for acquiring point cloud data of a region to be massaged in a target object;

a first calculation module configured for determining a proportion coefficient according to the two-dimensional data of the preliminary massage-point position and the point cloud data of the region to be massaged in the target object, and determining the two-dimensional data of target massage-point positions based on the two-dimensional data of the preliminary massage-point position and the proportion coefficient;

a second calculation module configured for acquiring target three-dimensional massage point position data according to the two-dimensional data of target massage-point positions and the point cloud data of the region to be massaged in the target object;

a third calculation module configured for generating a final massage track based on a preset massage track planning algorithm according to the target three-dimensional massage point position data;

wherein the preset massage track planning algorithm comprises grouping target three-dimensional massage-point positions according to a preset grouping rule to obtain a plurality of massage point position groups;

generating a plurality of segments of massage tracks according to the plurality of massage point position groups; and splicing the plurality of segments of massage tracks to generate a final massage track. (III) Beneficial effects

The above technical solution of the present invention has the following advantageous technical effects.

The present invention may select a massage mode, and there are different two-dimensional data of preliminary massage-point positions according to different massage modes so as to improve individual adaptability. According to the individual differences, the two-dimensional data of the massage point positions of the target are obtained to further improve the individual adaptability, and also provide a basis for further massage track planning. The preset massage track planning algorithm reduces the amount of calculation and the planned massage track is smooth, which is conducive to promotion.

**Brief Description of the Drawings**

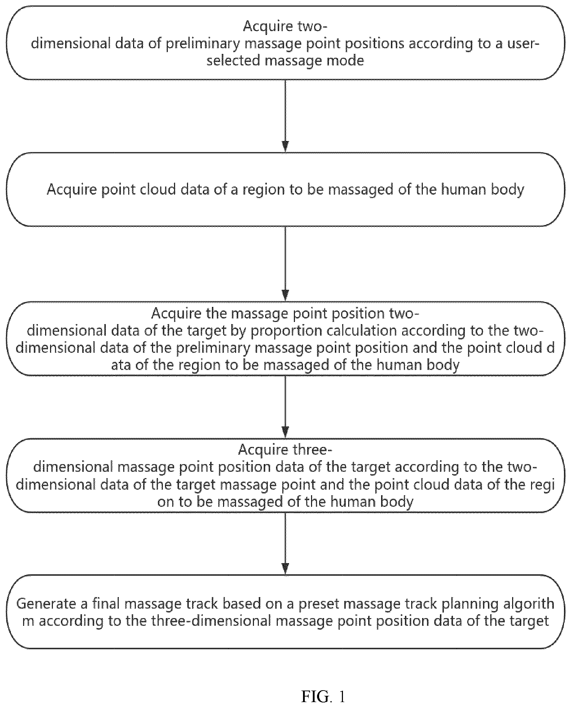

Fig. 1 is an overall flow diagram of an example of the present invention.

**Detailed Description of the Invention**

The objects, technical solution, and beneficial effects of the present invention will become more apparent from the detailed description set forth below when taken in conjunction with the drawings. It should be understood that the description is intended for purposes of illustration only, and is not intended to limit the scope of the present invention. Further, in the following description, descriptions of well-known structures and techniques are omitted to avoid unnecessarily obscuring the concepts of the present invention.

Obviously, the described examples are some, but not all, examples of the present invention. Based on the examples in the invention, all other examples obtained by a person skilled in the art without involving any inventive effort are within the scope of protection of the invention.

In the description of the present invention, it should be noted that, the terms "first", "second" and "third" are used solely for descriptive purposes and are not to be construed as indicating or implying relative importance.

Furthermore, the technical features involved in the various embodiments of the invention described below may be combined with each other as long as they do not conflict with each other.

In the following description, numerous specific details are set forth, such as device structures, materials, dimensions, processing techniques and techniques, in order to provide a more clear understanding of the present invention. However, it will be understood by those skilled in the art that the present invention may be practiced without these specific details.

**Example 1**

According to a method for generating a massage track, as shown in Fig. 1, an overall flow chart of an example of the present invention comprises:

Taking the human body massage as an example, two-dimensional data of preliminary massage-point positions is acquired according to a selected massage mode. The method selected here can be a method selected by a user or recommended by large data. The specific content of the massage mode is not limited here, and may be for whole body, half-body, abdomen, or back. Alternatively, in the present example, the massage mode covers full-back massage point positions, a waist massage point, a shoulder massage point and an abdomen massage point, etc. and a standard massage scheme can be quickly formed. In addition, it also supports user-defined massage point positions, such as "Ashi point" in traditional Chinese medicine, with higher flexibility and adaptability. After selecting a massage mode, the two-dimensional data of the preliminary massage-point position is correspondingly acquired in the corresponding massage mode.

The point cloud data of a region to be massaged in a target object is acquired. The specific method for acquiring the point cloud data of the region to be massaged in the target object is not limited herein. Optionally, a camera is used for photographing. The specific content of the camera is not limited herein, and the specific method for photographing is also not limited herein. Optionally, in the present example, the camera may be a depth camera, and the massage robot is controlled to move to a photographing pose for photographing. Optionally, in the present example, a person lies on or lies on a massage bed, and the massage robot is controlled to move above the target object for photographing. In the present example, the point cloud data of the region to be massaged in the target object may be directly acquired by using the depth camera for photographing.

A proportion coefficient is determined according to the two-dimensional data of the preliminary massage-point position and the point cloud data of the region to be massaged in the target object, and the two-dimensional data of target massage-point positions is determined based on the two-dimensional data of the preliminary massage-point position and the proportion coefficient. The two-dimensional data of the preliminary massage-point position is used as a reference, and the point cloud data of the region to be massaged in the target object is acquired according to an individual. A proportion coefficient is determined according to the two-dimensional data of the preliminary massage-point position and the point cloud data of the region to be massaged in the target object. The two-dimensional data in the acquired point cloud data of the region to be massaged in the target object is used for calculation. For example, a person is fat, and the two-dimensional data of the preliminary massage-point position can be expanded to obtain the target massage point position two-dimensional data. According to the change of the point cloud data, the density of the points taken of the massage point positions on the massage track is determined.

The target three-dimensional massage point position data is acquired according to the two-dimensional data of target massage-point positions and the point cloud data of the region to be massaged in the target object. The specific content of the target three-dimensional massage point position data is not limited here. Optionally, in the present example, the target three-dimensional massage point position data comprises a three-dimensional coordinate value of the massage point position and a Z-axis direction vector, wherein the three-dimensional coordinate value comprises X-axis, Y-axis and Z-axis data. The target three-dimensional massage point position data is massage point position data adapted to the user himself. It is the basis for further planning the massage track.

A final massage track is generated based on a preset massage track planning algorithm according to the target three-dimensional massage point position data. The specific content of the preset massage track planning algorithm is not limited here, and a person skilled in the art would be able to understand that it works when the massage track planning can be performed. Optionally, in the present example, the generating a final massage track based on a preset massage track planning algorithm according to the target three-dimensional massage point position data comprises the followings.

The target three-dimensional massage point positions are grouped according to a preset grouping rule to obtain a plurality of massage point position groups; the specific content of the preset grouping rule is not defined herein, and may be an average score according to the number of target three-dimensional massage point positions, or may be an allocation according to the minimum number of units. A person skilled in the art would be able to understand that it works when the grouping can be completed. Alternatively, in the present example, the preset grouping rule comprises the followings. the number of massag-point positions is acquired according to the target three-dimensional massage point position data.

The minimum number of grouping units is preset. The specific number of the minimum number of units of grouping is not limited here. Optionally, in the present example, the preset minimum number of units of grouping is 7, here the preset minimum number of units of grouping is 7 because 7 is the minimum number of points for realizing smooth concatenation, and the number of points is the minimum, so as to ensure that the matrix inversion is the simplest within the range meeting the requirements to simplify the calculation.

It is judged whether the number of massage-point positions is greater than a preset minimum number of grouping units. Here, firstly, it is judged whether the number of massage-point positions is greater than 7.

If the number of massage-point positions is greater than a preset minimum number of grouping units, successively sliding a frame to take points for grouping according to the preset minimum number of grouping units until the number of massage-point positions of the last group is less than or equal to the preset minimum number of grouping units. Here, the specific contents of successively sliding the frame to take points for the grouping are a first group 1-7, a second **group 2-8** and a third group 3-9, and so on successively. The number of points in each group is 7, the last group is determined by the number of points, and is not greater than 7 points.

If the number of massage-point positions is less than or equal to the preset minimum number of grouping units, no grouping is required. The grouping can generate a plurality of segments of massage tracks, and the plurality of segments of massage tracks are spliced, and the plurality of massage track formed by the grouping can ensure a smooth transition at the splice.

The key points of massage point positions are selected in a plurality of massage point position groups respectively. Alternatively, in the present example, it may be the massage point positions that must be passed during the massage process. The number of key points is not limited herein. Alternatively, in the present example, the number of key points in each massage point position group is not greater than the number of massage-point positions in the corresponding massage point position group.

The node vectors of a plurality of massage point position groups based on the key points are respectively calculated according to a preset first calculation method, wherein a first and a last node repetition degree is preset in the first calculation method. The preset first-end node repetition degree is not limited here. Optionally, in the present example, the preset first-end node repetition degree is 4. The repetition degree of 4 is to ensure that the first and last points of each massage point position group is at the key point, so that the repetition degree of the first and last nodes is set as (3 + 1). Optionally, in the present example, the preset first calculation method comprises:

presetting a first-end node repetition degree to 4;

$$u_0 = u_1 = u_2 = u_3 = 0$$

$$u_{i+3} = u_{i+2} + \frac{|p_i - p_{i-1}|}{\sum_{t=1}^{n}|p_t - p_{t-1}|}$$

$$u_{n+2} = u_{n+3} = u_{n+4} = u_{n+5} = 1$$

wherein the node vector is represented as $U = [u_0, u_1, ..., u_{n+5}]$; n represents the number of key points; i represents a sequence number of the current node; $p_i$: an $i^{th}$ key point; t: a sequence number of summation of all key points; and pt: a $t^{th}$ key point. The existence of repeated nodes can only provide n linear equations for n + 2 unknown control vertices, so that two boundary conditions should be added to both ends of the curve to make the number of equations equal to the number of unknown control vertices. Thus, the control vertices can be solved;

The boundary conditions of a plurality of massage point position groups are preset. The specific contents of boundary conditions are not limited here. Optionally, the boundary conditions are a tangent vector condition, a free end point condition and a closed curve condition, etc. wherein the tangent vector condition requires that the tangential direction is fixed, the free end point condition is suitable for the case where the curvature of the first end point is zero, and the closed curve condition is suitable for the case where the first and last points are overlapped and are second-order continuous. Furthermore, in the present example, the free end point condition is used as the boundary condition to ensure that the continuity of the curve at the first end point is optimal.

According to the node vector and the preset boundary condition, the control vertices of the plurality of massage point position groups are respectively calculated according to a preset second calculation method. Optionally, in the present example, the preset second calculation method includes:

obtaining a control vertex by calculating an inverse matrix of a following square matrix;

$$\begin{bmatrix} 1 & & & & & \\ a_2 & b_2 & c_2 & & & \\ & \bullet & \bullet & \bullet & & \\ & & \bullet & \bullet & \bullet & \\ & & & \bullet & \bullet & \bullet \\ & & & a_{n-1} & b_{n-1} & c_{n-1} \\ & & & & & 1 \end{bmatrix} \begin{bmatrix} d_1 \\ d_2 \\ \bullet \\ \bullet \\ \bullet \\ d_{n-1} \\ d_n \end{bmatrix} = \begin{bmatrix} e_1 \\ e_2 \\ \bullet \\ \bullet \\ \bullet \\ e_{n-1} \\ e_n \end{bmatrix}$$

where $d_i$ is a control vertex; and $a_i, b_i, c_i, e_i$ are all parameters related to key points and node vectors; the control top point can be obtained by matrix operation; and the inverse of the previous square matrix is required to find the control vertex. The calculation of the inverse matrix is very cumbersome. When the order is higher, the calculated amount of the inverse matrix doubles. Therefore, with regard to the track planning of the massage point positions with too many points in the present example, it is necessary to perform grouping processing in advance, respectively perform massage track planning on each group of points, and finally perform multi-segment massage track splicing to obtain a smooth massage track.

generating a plurality of segments of massage tracks according to the control vertices of the plurality of massage point position groups according to a preset third calculation method. The specific content of the preset third calculation method is not limited here. Optionally, in the present example, the preset third calculation method comprises generating a massage track according to a node vector, a control vertex and a weight factor;

$$p(u) = \frac{\sum\limits_{i=0}^{n} \omega_i d_i N_{i,k}(u)}{\sum\limits_{i=0}^{n} \omega_i N_{i,k}(u)}$$

where u represents a node parameter and an argument $\in [0, 1]$; $d_i$ represents a control vertex; k represents the order of a curve; $N_{i,k}(u)$ represents a $k^{th}$ B-spline fundamental function; and $\omega_i$ represents a weight factor; wherein the B-spline fundamental function formulation comprises:

$$N_{i,0}(u) = \begin{cases} 1, & u_i \leq u < u_{i+1} \\ 0, & \text{others} \end{cases}$$

$$N_{i,k}(u) = \frac{u - u_i}{u_{i+k} - u_i} N_{i,k-1}(u) + \frac{u_{i+k+1} - u}{u_{i+k+1} - u_{i+1}} N_{i+1,k-1}(u)$$

setting

$$\frac{0}{0} = 0$$

where $N_{i,k}(u)$ represents a $k^{th}$ B-spline fundamental function; and u represents a node parameter;
splicing the plurality of segments of massage tracks to generate a preliminary massage track.

In an example of the present invention, further, the generating a final massage track based on a preset massage track planning algorithm according to the target three-dimensional massage point position data further includes:
according to the target three-dimensional massage point position data in the preliminary massage track, calculating the pose of each target three-dimensional massage point position according to a preset pose algorithm, and generating a final massage track. Furthermore, optionally, in the present example, the preset pose algorithm includes:

according to the target three-dimensional massage point position data, the three-dimensional massage point position data comprises a three-dimensional coordinate value and a Z-axis direction vector of the massage point position; the three-dimensional coordinate value comprises X-axis, Y-axis and Z-axis data;
acquiring an X-axis direction vector of each three-dimensional massage point position of the target according to data of each target three-dimensional massage point position in the preliminary massage track;
acquiring a Y-axis direction vector according to a cross product of the X-axis and Z-axis direction vectors of each target three-dimensional massage point positions;
correcting the Z-axis direction vector by using a three-axis pair-wise perpendicular manner according to the direction vectors of the X-axis and Y-axis; and
obtaining the pose of each target three-dimensional massage point position, and generating a final massage track.

The present example can select a massage mode, comprising a self-defined mode, and having different two-dimensional data of preliminary massage-point positions according to different massage modes so as to improve individual adaptability. The two-dimensional data of target massage point positions is obtained according to individual differences, further improving individual adaptability, and also providing a basis for further massage track planning. According to the preset massage track planning algorithm, the number of massage-point positions are grouped, the grouping is made according to the minimum number of points for smooth splicing, and the number of points is the minimum, so as to ensure that the matrix inversion is the simplest within the range meeting the requirements to simplify the calculation. The multi-segment planning massage track, and then splicing are made to achieve smooth transition of massage tracks, and to save computing power to the greatest extent. It is conducive to promotion.

**Example 2**

A system for generating a massage track includes:

a selecting module configured for acquiring two-dimensional data of preliminary massage-point positions according to a selected massage mode; here, the specific content of the massage mode is not limited here, and may be for whole body, half-body, abdomen, or back; alternatively, in the present example, the massage mode covers full-back massage point positions, a waist massage point, a shoulder massage point and an abdomen massage point, etc. and a standard massage scheme can be quickly formed; in addition, it also supports user-defined massage point positions, such as "Ashi point" in traditional Chinese medicine, with higher flexibility and adaptability; after selecting a massage mode, the two-dimensional data of the preliminary massage-point position is correspondingly acquired in the corresponding massage mode;

an acquisition module configured for acquiring the point cloud data of a region to be massaged in a target object; here, the specific method for acquiring the point cloud data of the region to be massaged in the target object is not limited herein; optionally, a camera is used for photographing; the specific content of the camera is not limited herein, and the specific method for photographing is also not limited herein; optionally, in the present example, the camera may be a depth camera, and the massage robot is controlled to move to a photographing pose for photographing; optionally, in the present example, a person lies on or lies on a massage bed, and the massage robot is controlled to move above the target object for photographing; and in the present example, the point cloud data of the region to be massaged in the target object may be directly acquired by using the depth camera for photographing.

a first calculation module configured for determining a proportion coefficient according to the two-dimensional data of the preliminary massage-point position and the point cloud data of the region to be massaged in the target object, and determining the two-dimensional data of target massage-point positions based on the two-dimensional data of the preliminary massage-point position and the proportion coefficient; the two-dimensional data of the preliminary massage-point position is used as a reference, and the point cloud data of the region to be massaged in the target object is acquired according to an individual, and the two-dimensional data of target massage-point positions is acquired by calculating according to a proportion; for example, a person is fat, and the two-dimensional data of the preliminary massage-point position can be expanded to obtain the target massage point position two-dimensional data. According to the change of the point cloud data, the density of the points taken of the massage point positions on the massage track is determined.

a second calculation module configured for acquiring the target three-dimensional massage point position data according to the two-dimensional data of target massage-point positions and the point cloud data of the region to be massaged in the target object; here, the specific content of the target three-dimensional massage point position data is not limited; optionally, in the present example, the target three-dimensional massage point position data comprises a three-dimensional coordinate value of the massage point position and a Z-axis direction vector, where the three-dimensional coordinate value comprises X-axis, Y-axis and Z-axis data; the target three-dimensional massage point position data is massage point position data adapted to the user himself; it is the basis for further planning the massage track; and

a third calculation module configured for generating a final massage track based on a preset massage track planning algorithm according to the target three-dimensional massage point position data. The specific content of the preset massage track planning algorithm is not limited here, and a person skilled in the art would be able to understand that it works when the massage track planning can be performed. Optionally, in the present example, this example comprises:

grouping target three-dimensional massage-point positions according to a preset grouping rule to obtain a plurality of massage point position groups;
selecting key points of massage point positions in a plurality of massage point position groups respectively;
calculating node vectors of a plurality of massage point position groups respectively, based on the key points according to a preset first calculation method, wherein a first and a last node repetition degree is preset in the first calculation method;
presetting boundary conditions of a plurality of massage point position groups;
according to the node vector and the preset boundary condition, calculating control vertices of the plurality of massage point position groups respectively, according to a preset second calculation method;
generating a plurality of segments of massage tracks according to the control vertices of the plurality of massage point position groups according to a preset third calculation method; and
splicing the plurality of segments of massage tracks to generate a preliminary massage track.

Furthermore, in the present example, the generating a final massage track based on a preset massage track planning algorithm according to the target three-dimensional massage point position data further comprises:
according to the target three-dimensional massage point position data in the preliminary massage track, calculating the pose of each target three-dimensional massage point position according to a preset pose algorithm, and generating a final massage track.

It should be understood that the above-described examples of the invention are merely illustrative of or explanatory of the principles of the invention, and are not restrictive of the invention. Accordingly, it is intended to embrace all such alternatives, modifications, and variations that fall within the spirit and scope of the present invention. Furthermore, it is intended that the appended claims cover all such variations and modifications as come within the scope and range of equivalents of the claims appended hereto.

The invention has been described above with reference to examples thereof. However, these examples are for illustrative purposes only and are not intended to limit the scope of the present invention. It is intended that the scope of the invention be defined by the claims appended hereto and their equivalents. Various alternatives and modifications can be devised by those skilled in the art without departing from the scope of the invention, and these alternatives and modifications are intended to be included within the scope of the invention.

Although embodiments of the present invention have been described in detail, it should be understood that various changes, substitutions and alterations can be made herein without departing from the spirit and scope of the invention.

Obviously, the above-described embodiments are merely illustrative for clarity of the examples given and are not restrictive of the implementations. It will be apparent to those skilled in the art that various other modifications and variations can be made in the invention without departing from the scope or spirit of the invention. All implementations need not be, and cannot be, exhaustive. The obvious changes or alterations resulting therefrom are still within the scope of protection of the invention.

Those skilled in the art will appreciate that embodiments of the present invention may be provided as a method, a system, or a computer program product. Thus, the present invention may take the form of an entire hardware embodiment, an entire software embodiment, or an embodiment combining software and hardware aspects. Moreover, the present invention may take the form of a computer program product embodied on one or more computer-usable storage media (including, but not limited to, magnetic disk storage, CD-ROM, optical storage, and the like) having computer-usable program code embodied therein.

**Claims**

1. A method for generating a massage track, comprising:

   acquiring two-dimensional data of preliminary massage-point positions according to a selected massage mode;
   acquiring point cloud data of a region to be massaged in a target object;
   determining a proportion coefficient according to the two-dimensional data of the preliminary massage-point position and the point cloud data of the region to be massaged in the target object, and determining two-dimensional data of target massage-point positions based on the two-dimensional data of the preliminary massage-point positions and the proportion coefficient;
   acquiring target three-dimensional massage point position data according to the two-dimensional data of target massage-point positions and the point cloud data of the region to be massaged in the target object; and
   generating a final massage track based on a preset massage track planning algorithm according to the target three-dimensional massage point position data;
   wherein the preset massage track planning algorithm comprises grouping target three-dimensional massage-point positions according to a preset grouping rule to obtain a plurality of massage point position groups;
   generating a plurality of segments of massage tracks according to the plurality of massage point position groups; and
   splicing the plurality of segments of massage tracks to generate a final massage track.

2. The method for generating the massage track according to claim 1, wherein the generating a final massage track based on a preset massage track planning algorithm according to the target three-dimensional massage point position data further comprises:

   selecting key points of massage point positions in a plurality of massage point position groups respectively;
   calculating node vectors of a plurality of massage point position groups respectively, based on the key points according to a preset first calculation method;
   presetting boundary conditions of a plurality of massage point position groups;
   according to the node vector and the preset boundary condition, calculating control vertices of the plurality of massage point position groups respectively, according to a preset second calculation method;
   generating a plurality of segments of massage tracks according to the control vertices of the plurality of massage point position groups according to a preset third calculation method; and

splicing the plurality of segments of massage tracks to generate a preliminary massage track.

3. The method for generating the massage track according to claim 2, wherein the generating a final massage track based on a preset massage track planning algorithm according to the target three-dimensional massage point position data further comprises:
according to the target three-dimensional massage point position data in the preliminary massage track, calculating the pose of each target three-dimensional massage point position according to a preset pose algorithm, and generating a final massage track.

4. The method for generating the massage track according to claim 2, wherein the preset grouping rule comprises:

acquiring the number of massag-point positions according to the target three-dimensional massage point position data;
presetting a minimum number of the grouping units;
judging whether the number of massage-point positions is greater than a preset minimum number of grouping units;
if the number of massage-point positions is greater than a preset minimum number of grouping units, successively sliding a frame to take points for grouping according to the preset minimum number of grouping units, until the number of massage-point positions of the last group is less than or equal to the preset minimum number of grouping units; and
if the number of massage-point positions is less than or equal to the preset minimum number of grouping units, no grouping is required.

5. The method for generating the massage track according to claim 2, wherein the preset first calculation method comprises:

presetting a first-end node repetition degree to 4;

$$u_0 = u_1 = u_2 = u_3 = 0$$

$$u_{i+3} = u_{i+2} + \frac{|p_i - p_{i-1}|}{\sum_{t=1}^{n}|p_t - p_{t-1}|}$$

$$u_{n+2} = u_{n+3} = u_{n+4} = u_{n+5} = 1$$

wherein the node vector is represented as $U = [u_0, u_1, ..., u_{n+5}]$; n represents the number of key points; i represents a sequence number of the current node; $p_i$: an $i^{th}$ key point; t: a sequence number of summation of all key points; and pt: a $t^{th}$ key point.

6. The method for generating the massage track according to claim 2, wherein the preset second calculation method comprises:

obtaining a control vertex by calculating an inverse matrix of a following square matrix;

$$\begin{bmatrix} 1 & & & & & \\ a_2 & b_2 & c_2 & & & \\ & \bullet & \bullet & \bullet & & \\ & & \bullet & \bullet & \bullet & \\ & & & \bullet & \bullet & \bullet \\ & & & a_{n-1} & b_{n-1} & c_{n-1} \\ & & & & & 1 \end{bmatrix} \begin{bmatrix} d_1 \\ d_2 \\ \bullet \\ \bullet \\ \bullet \\ d_{n-1} \\ d_n \end{bmatrix} = \begin{bmatrix} e_1 \\ e_2 \\ \bullet \\ \bullet \\ \bullet \\ e_{n-1} \\ e_n \end{bmatrix}$$

where $d_i$ is a control vertex; and $a_i, b_i, c_i, e_i$ are all parameters related to key points and node vectors.

7. The method for generating the massage track according to claim 2, wherein the preset third calculation method comprises:

$$p(u) = \frac{\sum_{i=0}^{n} \omega_i d_i N_{i,k}(u)}{\sum_{i=0}^{n} \omega_i N_{i,k}(u)}$$

where u represents a node parameter and an argument $\in$ [0, 1]; $d_i$ represents a control vertex; k represents the order of a curve; $N_{i,k}(u)$ represents a $k^{th}$ B-spline fundamental function; and $\omega_i$ represents a weight factor.

8. The method for generating the massage track according to claim 7, wherein the B-spline fundamental function formulation comprises:

$$N_{i,0}(u) = \begin{cases} 1, & u_i \leq u < u_{i+1} \\ 0, & \text{others} \end{cases}$$

$$N_{i,k}(u) = \frac{u - u_i}{u_{i+k} - u_i} N_{i,k-1}(u) + \frac{u_{i+k+1} - u}{u_{i+k+1} - u_{i+1}} N_{i+1,k-1}(u)$$

setting

$$\frac{0}{0} = 0$$

where $N_{i,k}(u)$ represents a $k^{th}$ B-spline fundamental function; and u represents a node parameter.

9. The method for generating the massage track according to claim 3, wherein the preset pose algorithm comprises:

according to the target three-dimensional massage point position data, the three-dimensional massage point position data comprises a three-dimensional coordinate value and a Z-axis direction vector of the massage point position; the three-dimensional coordinate value comprises X-axis, Y-axis and Z-axis data;
acquiring an X-axis direction vector of each three-dimensional massage point position of the target according to data of each target three-dimensional massage point position in the preliminary massage track;
acquiring a Y-axis direction vector according to a cross product of the X-axis and Z-axis direction vectors of each target three-dimensional massage point positions;
correcting the Z-axis direction vector by using a three-axis pair-wise perpendicular manner according to the direction vectors of the X-axis and Y-axis; and
obtaining the pose of each target three-dimensional massage point position, and generating a final massage track.

10. A system for generating a massage track, comprising:

a selecting module configured for acquiring two-dimensional data of preliminary massage-point positions according to a selected massage mode;
an acquisition module configured for acquiring point cloud data of a region to be massaged in a target object;
a first calculation module configured for determining a proportion coefficient according to the two-dimensional data of the preliminary massage-point position and the point cloud data of the region to be massaged in the target object, and determining the two-dimensional data of target massage-point positions based on the two-dimensional data of the preliminary massage-point position and the proportion coefficient;
a second calculation module configured for acquiring target three-dimensional massage point position data according to the two-dimensional data of target massage-point positions and the point cloud data of the region to be massaged in the target object;

a third calculation module configured for generating a final massage track based on a preset massage track planning algorithm according to the target three-dimensional massage point position data;

wherein the preset massage track planning algorithm comprises grouping target three-dimensional massage-point positions according to a preset grouping rule to obtain a plurality of massage point position groups;

generating a plurality of segments of massage tracks according to the plurality of massage point position groups; and

splicing the plurality of segments of massage tracks to generate a final massage track.

Acquire two-dimensional data of preliminary massage point positions according to a user-selected massage mode

Acquire point cloud data of a region to be massaged of the human body

Acquire the massage point position two-dimensional data of the target by proportion calculation according to the two-dimensional data of the preliminary massage point position and the point cloud data of the region to be massaged of the human body

Acquire three-dimensional massage point position data of the target according to the two-dimensional data of the target massage point and the point cloud data of the region to be massaged of the human body

Generate a final massage track based on a preset massage track planning algorithm according to the three-dimensional massage point position data of the target

FIG. 1

## INTERNATIONAL SEARCH REPORT

International application No.

**PCT/CN2023/140192**

**A. CLASSIFICATION OF SUBJECT MATTER**

G06F17/10(2006.01)i; G06T7/20(2017.01)i; G06F17/16(2006.01)i; A61H7/00(2006.01)i

According to International Patent Classification (IPC) or to both national classification and IPC

**B. FIELDS SEARCHED**

Minimum documentation searched (classification system followed by classification symbols)

IPC: G06F, G06T, A61H

Documentation searched other than minimum documentation to the extent that such documents are included in the fields searched

Electronic data base consulted during the international search (name of data base and, where practicable, search terms used)

CNTXT, ENTXT, ENTXTC, DWPI, CNKI: 按摩, 轨迹, 二维, 三维, 云数据, 点, 调整, 改变, 初始, massage, track, 2D, 3D, two-dimensional, three-dimensional, cloud data, dot, point, adjust, change

**C. DOCUMENTS CONSIDERED TO BE RELEVANT**

| Category* | Citation of document, with indication, where appropriate, of the relevant passages | Relevant to claim No. |
|---|---|---|
| PX | CN 116932979 A (REALMAN INTELLIGENT TECHNOLOGY (BEIJING) CO., LTD.) 24 October 2023 (2023-10-24) <br> entire document | 1-10 |
| X | CN 113506604 A (AUBO (BEIJING) ROBOTICS TECHNOLOGY CO., LTD.) 15 October 2021 (2021-10-15) <br> description, paragraphs 93-149 | 1, 10 |
| A | CN 113506604 A (AUBO (BEIJING) ROBOTICS TECHNOLOGY CO., LTD.) 15 October 2021 (2021-10-15) <br> entire document | 2-9 |
| A | CN 108466265 A (ZHUHAI JUNKAI MACHINERY TECHNOLOGY CO., LTD.) 31 August 2018 (2018-08-31) <br> entire document | 1-10 |
| A | CN 115741732 A (FUZHOU UNIVERSITY) 07 March 2023 (2023-03-07) <br> entire document | 1-10 |
| A | KR 102378105 B1 (INNOSIMULATION CO., LTD.) 24 March 2022 (2022-03-24) <br> entire document | 1-10 |

☐ Further documents are listed in the continuation of Box C.   ☑ See patent family annex.

| | |
|---|---|
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **25 January 2024** | **02 February 2024** |

| Name and mailing address of the ISA/CN | Authorized officer |
|---|---|
| **China National Intellectual Property Administration (ISA/ CN)** <br> **China No. 6, Xitucheng Road, Jimenqiao, Haidian District, Beijing 100088** | |
| | Telephone No. |

Form PCT/ISA/210 (second sheet) (July 2022)

<table>
<tr><td colspan="2" align="center">INTERNATIONAL SEARCH REPORT<br>Information on patent family members</td><td colspan="2">International application No.<br><br>**PCT/CN2023/140192**</td></tr>
</table>

| Patent document cited in search report | | | Publication date (day/month/year) | Patent family member(s) | | | Publication date (day/month/year) |
|---|---|---|---|---|---|---|---|
| CN | 116932979 | A | 24 October 2023 | CN | 116932979 | B | 26 December 2023 |
| CN | 113506604 | A | 15 October 2021 | CN | 113506604 | B | 12 July 2022 |
| CN | 108466265 | A | 31 August 2018 | CN | 108466265 | B | 31 August 2017 |
| CN | 115741732 | A | 07 March 2023 | None | | | |
| KR | 102378105 | B1 | 24 March 2022 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311200597X